# FASCICULE DE BREVET EUROPEEN

(11) **EP 1 871 854 B1**
(45) Date de publication et mention de la délivrance du brevet: **20.10.2010**
(21) Numéro de dépôt: 06755425.3
(22) Date de dépôt: 19.04.2006
(51) Int. Cl.: C09J 153/02

(54) **COMPOSITION ADHÉSIVE ET ÉLÉMENT DE FIXATION SUR LA PEAU HUMAINE**
KLEBSTOFFZUSAMMENSETZUNG UND ELEMENT ZUM AUFKLEBEN AUF MENSCHLICHE HAUT
ADHESIVE COMPOSITION AND ELEMENT FOR ATTACHING TO HUMAN SKIN

(30) Priorité: 19.04.2005 FR 0503903
(43) Date de publication de la demande: 02.01.2008
(73) Titulaire: B.Braun Medical SAS, 92100 Boulogne-Billancourt (FR)
(72) Inventeur: LASSALLE, Paul, F-64200 BIARRITZ (FR)
(74) Mandataire: Schmit, Christian Norbert Marie
(86) Numéro de dépôt international: PCT/FR2006/000860
(87) Numéro de publication internationale: WO 2006/111654

(56) Documents cités:
- WO-A-00/54820
- US-A- 5 492 943
- US-A1- 2003 225 356
- US-B1- 6 326 421
- US-B1- 6 458 886

## Description

L'invention concerne une composition adhésive destinée essentiellement à constituer un élément adhésif par exemple pour poches de recueil de fluides corporels, telles que des poches de stomie ou des éléments analogues tels que des pansements.

On utilise dans le présent mémoire la terminologie suivante.

Une "composition adhésive", notamment pour poche de recueil de fluides corporels, comporte une "phase continue" à base de polymères, élastomères, etc. et une "phase discontinue" contenant essentiellement des hydrocolloïdes.

La phase continue contient différents copolymères, polymères, élastomères qui ont une fonction de création d'une texture, éventuellement des plastifiants qui ont une fonction d'augmentation de souplesse de la composition, et des résines d'adhésivité, parfois désignées par le néologisme "tackifiant" qui correspond au terme anglais "tackifier".

Cette terminologie, qui distingue les ingrédients de texture (élastomères, polymères, copolymères) des plastifiants et des agents d'adhésivité ne doit pas être considérée comme rigoureuse. En effet, la plupart des polymères, copolymères et élastomères ont plus ou moins des propriétés de texture (à moins qu'ils ne soient liquides), de plastifiant et d'agent d'adhésivité en présence d'autres ingrédients. Il faut donc considérer l'action de ces ingrédients dans le cadre des compositions adhésives utilisées pour la fixation, directe ou indirecte, des poches de recueil de fluides corporels. Cependant, cette terminologie permet de distinguer les différentes compositions adhésives utilisées pour la fixation des poches de recueil de fluides corporels.

Les compositions adhésives pour poches de recueil de fluides corporels sont utilisées pour la fixation d'une poche par collage direct à la peau d'un être humain, autour d'un orifice d'évacuation d'excréments. Le collage peut être prévu pour durer un temps relativement court, qui peut être inférieur à une journée, ou un temps relativement important, qui peut être de l'ordre d'une semaine. Les compositions adhésives utilisées pour ces diverses applications ont donc des propriétés différentes, selon qu'elles doivent rester fixées pendant un temps plus ou moins long. Cependant, elles ont toutes un certain nombre de propriétés communes.

Les compositions adhésives de fixation des poches de recueil de fluides corporels doivent d'abord posséder certaines propriétés mécaniques. Ces propriétés mécaniques sont essentiellement d'abord la tenue mécanique d'une poche, qui peut avoir un certain poids, sans décollement de la composition adhésive de la peau, ensuite l'absence de fuite au niveau de la composition adhésive, et enfin une bonne adaptabilité destinée à donner un bon confort qui est non seulement une bonne commodité à la mise en place et à l'enlèvement, mais surtout une souplesse suffisante pour que le patient oublie autant que possible la présence de la composition adhésive et de la poche.

Les compositions adhésives de fixation des poches de recueil de fluides corporels doivent ensuite posséder certaines propriétés chimiques. Il faut essentiellement que la composition adhésive ne provoque pas une irritation cutanée.

Les premières compositions adhésives qui ont été utilisées de façon importante dans les années 1960 étaient à base de polybutène (phase continue) et d'hydrocolloïdes. Les quantités des deux ingrédients étaient très semblables. Les problèmes posés par ces compositions adhésives étaient essentiellement le fait que les polybutènes ne sont pas de bons polymères de texture et présentent facilement des phénomènes de dissolution et de délitement conduisant à la formation de fuites.

Un exemple de composition ayant des quantités réduites de polybutènes avec un copolymère séquencé de styrène est décrit dans le document US-5 492 943, mais pose aussi le problème précité de dissolution et de délitement.

On a proposé aussi, comme l'indique le brevet français FR-2 392 076, une composition adhésive sous forme d'un gel contenant au moins un élastomère à réticulation physique constituant la phase continue dans laquelle sont dispersés des hydrocolloïdes. En particulier, certaines compositions contiennent environ 10 % en poids de copolymères séquencés de styrène-isoprène-styrène ou de styrène-butadiène-styrène essentiellement à base de séquences triples, 16,7 à 20,6 % d'agents d'adhésivité appelés "résines poisseuses", 18 à 25 % d'un ingrédient liquide jouant le rôle d'un plastifiant, et 45,5 à 52 % d'hydrocolloïdes. Ces compositions étaient utilisées avec une épaisseur de l'ordre de 3 mm afin qu'elles possèdent les propriétés nécessaires non seulement d'adhésivité mais aussi de commodité de pose et d'enlèvement. Bien que présentant des propriétés bien meilleures au point de vue de la résistance au délitement que les compositions antérieures connues, les dispositifs de fixation de poche réalisés avec cette composition adhésive étaient relativement rigides et ne donnaient qu'un confort réduit.

De même, on a essayé d'utiliser ces polymères en proportion importante avec une quantité élevée de charge minérale, plus précisément des particules d'argile, comme décrit dans le document US2003/0225356. Cependant, les dispositifs de fixation réalisés avec cette composition adhésive ne donnaient aussi qu'un confort réduit.

On a donc considéré de façon générale que l'utilisation des copolymères de type styrène-isoprène-styrène, tels que le "Kraton", comme seul polymère de texture, posait des problèmes de rigidité : les compositions préparées ne conviennent que pour un port très long, nécessitant des propriétés exceptionnelles de texture. Ainsi, le document "Skin Barriers for Stoma Care" du Dr Kenji Tazawa, 2001, Alcare Co., Ltd. (Tokyo), indique les mauvais résultats obtenus avec de tels copolymères de texture.

C'est la raison pour laquelle ces copolymères styrène-isoprène-styrène ont été utilisés soit avec des copolymères séquencés de même type mais ayant des séquences doubles en grande quantité, à deux blocs seulement, soit avec des poly-isobutènes qui posent le problème précité de dissolution et de délitement, soit en association avec une couche de mousse.

Ainsi, la demande internationale de brevet WO 99/54422 décrit une composition adhésive sensible à la pression analogue à celle du brevet français précité FR-2 392 076, mais dans laquelle le polymère de texture est un copolymère séquencé essentiellement à base de séquences doubles, ayant une rigidité plus faible que celle du copolymère à séquences triples. La quantité de copolymère séquencé, dans toutes les compositions indiquées, est très importante et varie entre 18 et 40 %. Cette composition forme des éléments réalisés avec des épaisseurs de l'ordre de 1 mm pour être utilisés pour la fixation de poches de recueil de fluides corporels.

Le brevet européen EP-1 007 597 concerne aussi des matières adhésives comprenant une phase continue et une phase discontinue de polymères hydrophiles. La phase continue est constituée d'un mélange de caoutchouc liquide et de caoutchouc solide, le caoutchouc liquide étant prépondérant. La plage de quantité de la phase discontinue est extrêmement large (10 à 70 %). Ce document indique que l'avantage de cette composition adhésive est d'éviter l'utilisation des résines d'adhésivité (tackifier) considérée comme la cause de l'irritation cutanée. Un exemple indique cependant la présence d'une petite quantité (toujours inférieure à 12 %) d'une résine d'adhésivité à activité moyenne à réduite ("Regalite R91"). L'absence de "matériaux résineux" est préconisée.

L'invention concerne une composition adhésive qui, en contradiction avec les enseignements du dernier document cité, contient une quantité très importante d'agent d'adhésivité, introduite au détriment des hydrocolloïdes.

Plus précisément, pour une concentration de copolymère séquencé de type styrène-isoprène-styrène ou voisin analogue à celle de certaines compositions connues (inférieure à 10 %), la composition adhésive selon l'invention contient une quantité réduite d'hydrocolloïdes (25 à 45 %) et une quantité accrue d'agents d'adhésivité (plus de 22 %), notamment d'agents d'adhésivité ayant des propriétés d'adhésivité particulièrement fortes.

Plus précisément, l'agent d'adhésivité utilisé comprend un mélange d'agents d'adhésivité qui contient une majorité d'agents d'adhésivité de forte activité, à base de résines terpéniques, une petite quantité éventuelle d'un agent d'adhésivité à activité moyenne, et une quantité significative d'un agent d'adhésivité de faible activité d'adhésivité mais présentant en outre des propriétés renforçantes.

Le classement de l'activité des agents d'adhésivité comme étant "faible", "moyenne" ou "forte" est défini par un essai empirique couramment utilisé dans le domaine technique considéré. Dans cet essai, une composition de référence est préparée avec une quantité déterminée d'un copolymère défini et une quantité déterminée de la résine, dont les propriétés d'adhésivité doivent être déterminées, égale à la quantité du copolymère. Un essai normalisé d'arrachement de bille est ensuite exécuté dans une machine d'essais de traction et permet un classement des propriétés d'adhésivité d'après la force nécessaire à l'arrachement de la bille, dans des conditions déterminées de temps de contact avec la composition avant arrachement et de vitesse d'arrachement. Selon cet essai, une faible activité correspond à une force d'arrachement inférieure à 6 N, une activité moyenne à une force de 6 à 14 N, et une forte activité à une force de 14 à 25 N. Dans les exemples qui suivent, on utilise par exemple des agents d'adhésivité donnant des forces respectives de 4, 12 et 19 N. Dans les conditions indiquées, l'agent d'adhésivité cité dans le brevet européen précité EP-1 007 597 donne une force d'arrachement voisine de 6 N.

Avec les définitions données précédemment, une composition adhésive selon l'invention contient 55 à 75 % d'une phase continue et 25 à 45 % d'une phase discontinue constituée de polymères hydrophiles sous forme d'hydrocolloïdes, la phase continue contenant 5 à 10 % d'un copolymère de texture constitué d'un copolymère séquencé de type styrène-isoprène-styrène ou styrène-butadiène-styrène ayant une majorité de séquences triples au moins, plus de 22 % d'agent d'adhésivité, et 10 à 30 % d'un plastifiant, ces pourcentages étant indiqués par rapport à l'ensemble de la composition.

De préférence, la phase continue contient 6 à 10 % d'un copolymère de texture constitué d'un copolymère séquencé de type styrène-isoprène-styrène ou styrène-butadiène-styrène ayant une majorité de séquences triples au moins, 23 à 38 % d'agent d'adhésivité, et 13 à 25 % d'un plastifiant, ces pourcentages étant indiqués par rapport à l'ensemble de la composition.

Le copolymère de texture de la phase continue de type styrène-isoprène-styrène ou styrène-butadiène-styrène ayant une majorité de séquences triples au moins, est constitué de préférence d'un copolymère séquencé de styrène-isoprène-styrène composé entièrement ou en majorité de séquences triples au moins, les séquences doubles ne constituant qu'une minorité du copolymère.

Des exemples de copolymères de texture convenant selon l'invention sont par exemple certains copolymères "Kraton" de Shell Chemical Company, par exemple de type D11 (D1161, D1117 et D1112, ces deux derniers contenant respectivement 67 et 62 % de séquences triples au moins).

La moitié au moins de l'agent d'adhésivité est constituée d'un agent d'adhésivité de forte activité, à base de résine terpénique.

L'agent d'adhésivité de forte activité est avantageusement une résine polyterpénique de terpène-styrène, ayant avantageusement une température de ramollissement d'environ 105 °C (méthode à bille et anneau) et une masse moléculaire en masse de l'ordre de 1 000.

Un agent d'adhésivité est de faible activité et constitue une résine renforçante. De préférence, il constitue le tiers au maximum de l'agent d'adhésivité. Un tel agent d'adhésivité est avantageusement un polymère thermoplastique dérivé essentiellement de α-méthylstyrène ayant par exemple une masse moléculaire en masse comprise entre 300 et 3 000, tel que le polymère "Kristalex" F100 de Hercules. Une telle résine renforçante permet de moduler l'élasticité du copolymère à séquences triples, et empêche la migration des plastifiants, même lorsque ceux-ci sont présents en quantité importante.

Une quantité réduite de l'agent d'adhésivité peut être constituée d'un agent d'adhésivité d'activité faible à moyenne, tel que "Regalite" de Hercules qui est une résine totalement hydrogénée.

La phase discontinue constituée de polymères hydrophiles sous forme d'hydrocolloïdes en quantité comprise entre 25 à 45 % peut être de type bien connu dans le technique, contenant une quantité importante de composés tels que les fibres de cellulose, la carboxyméthylcellulose, sodique, réticulée ou autre, et l'hydroxyéthylcellulose, ainsi que des composés analogues à la gomme guar, et des substances telles que les xanthanes, les alginates, la pectine, la gélatine, le psyllium, l'extrait de caroube, la gomme arabique, l'agarose, les carraghénines et des polyacrylamides.

Les fibres de cellulose ont en particulier un rôle avantageux grâce à la création d'un réseau mécanique formé par l'enchevêtrement des fibres. Celui-ci augmente notablement la cohésion du mélange et limite ainsi les variations de dimensions lors du retrait dans les conditions d'utilisation, tout en favorisant la propagation de l'humidité dans la masse adhésive par un phénomène de capillarité.

Il est en outre avantageux que la composition adhésive comporte aussi un agent à action médicinale, par exemple choisi parmi le chitosane et le α-L-fucose.

L'invention concerne aussi un élément de fixation de poche de recueil de fluides corporels comprenant la composition adhésive précitée et, sur la face opposée à celle qui doit être au contact de la peau, un film de revêtement présentant une propriété de transmission unidirectionnelle d'eau, c'est-à-dire un film qui permet à l'eau absorbée par la composition adhésive, plus précisément des hydrocolloïdes que celle-ci contient, de s'évaporer en traversant par les microperforations vers l'extérieur et d'être ainsi évacuée.

Un tel film est un film de polyéthylène à microperforations conformées permettant l'évaporation de l'eau dans un sens mais interdisant le passage de l'eau dans l'autre sens.

On constate que la soudure réalisée entre d'une part un tel élément de fixation de poche de recueil de fluides corporels formé de la composition adhésive munie du film de polyéthylène et d'autre part la poche, autour du trou formé dans l'élément adhésif, garde une très grande souplesse et améliore donc le confort pour le patient.

### Exemples de composition

On prépare d'abord une composition (exemple 4) constituée d'un copolymère de type styrène-isoprène-styrène "Kraton" D1161BS (11,76 kg), d'une résine d'adhésivité "Regalite" R1100 (8,16 kg), d'une résine polyterpénique de terpène-styrène ayant une masse moléculaire d'environ 1 000 (17,64 kg), d'une résine d'adhésivité "Kristalex" F100 (11,76 kg), ayant aussi une action renforçante, et d'un agent plastifiant "Durasyn" 164 (24,72 kg), et on ajoute 6 kg de fibres de cellulose "BER400", puis 35,16 kg de "Cekol" et 4,8 kg de gomme guar HV225.

Après chaque introduction, une bonne homogénéité nécessite un malaxage de 10 min au moins. Le malaxage final dure 50 min et le temps d'extrusion est d'environ 60 min.

La composition ainsi obtenue est extrudée sous forme d'une feuille de 1 mm d'épaisseur et découpée en éléments utilisés pour des essais de fixation de poches de recueil de fluides corporels.

On prépare d'autres compositions selon le tableau qui suit.

| | 1 | 2 | 3 | 4 | 5 | 6 | 7 | 8 |
|---|---|---|---|---|---|---|---|---|
| Texture | | | | | | | | |
| "Kraton" D11 | 9,7 | | | 9,8 | | | | 9,2 |
| "Vector 4113" | | 9,7 | | | | 8,6 | 9,3 | |
| "Cariflex S-1707" | | | 9,8 | | 8,6 | | | |
| Résines | | | | | | | | |
| Forte activité | | | | | | | | |
| "Dercolyte" | | | | 14,7 | | | 16,2 | |
| "Piccolyte S" | 23,5 | 21,5 | | | | 15,7 | | 18,8 |
| "Zonatac" | | | 15,7 | | 15,7 | | | |
| Faible activité | | | | | | | | |
| "Regalite" R11 | 6,1 | | | 6,8 | 6,9 | | 10,0 | |
| "Arkon P" | | 6,1 | 7,8 | | | 7,9 | | 5,5 |
| Action renforçante | | | | | | | | |
| "Kristalex KF" | 9,7 | | | 9,8 | | | | |
| "Piccotex" | | | 9,8 | | | 8,6 | 9,3 | |
| "LX-685" | | 9,7 | | | 8,6 | | | 9,2 |
| Total résines | 39,3 | 37,3 | 33,3 | 31,3 | 31,2 | 32,2 | 35,5 | 33,5 |
| Plastifiant | 21,4 | 23,4 | 20,6 | 20,6 | 18,2 | 19,7 | 17,8 | 24,8 |
| "Durasyn" | | | | | | | | |
| Hydrocolloïdes | | | | | | | | |
| CMC "Cekol" | 15,9 | 15,9 | 21,3 | 29,3 | 29,5 | 25,0 | 19,3 | |
| Gomme karaya | | | | | | | 5,0 | |
| Fibres "BER400" | 4,0 | 4,0 | 3,0 | 5,0 | 4,0 | 3,0 | 4,5 | 4,5 |
| CMC "7MXF" | 4,7 | 4,7 | | | | 6,5 | | 12,0 |
| Gomme guar | 5,0 | | | 4,0 | | | | 7,0 |
| "Superabsorbant" | | 5,0 | 12,0 | | 8,5 | 5,0 | 8,6 | 8,0 |
| A500 | | | | | | | | |
| Chitosane | | | | | | | | 1,0 |
| Total hydrocolloïdes | 29,6 | 29,6 | 36,3 | 38,3 | 42,0 | 39,5 | 37,4 | 32,5 |
| Adhésivité | 5,0 | 4,8 | 4,2 | 4,0 | 4,7 | 4,6 | 4,7 | 4,2 |
| Module d'élasticité | 0,17 | 0,18 | 0,175 | 0,175 | 0,18 | 0,17 | 0,19 | 0,175 |
| Force maximale | 1,3 | 1,2 | 1,2 | 1,3 | 1,3 | 1,3 | 1,5 | 1,3 |
| Absorption | 100 | 150 | 225 | 185 | 350 | 220 | 190 | 70 |

Le terme "résines" désigne, de manière généralement admise dans la communauté scientifique et dans le présent mémoire, des composés dont la masse moléculaire en nombre est inférieure à 5 000, les composés de plus grande masse moléculaire étant appelés "polymères" ou "copolymères".

L'adhésivité est déterminée comme indiqué précédemment.

Le module d'élasticité et la force maximale d'arrachement sont définis par les méthodes normalisées d'essais mécaniques.

On note aussi que la plus faible adhésivité correspond au plus grand pourcentage de copolymère de texture, ce résultat confirmant l'analyse donnée au début du présent mémoire en référence au document précité "Skin Barriers for Stoma Care" du Dr Kenji Tazawa.

Le polymère de texture peut être, au lieu de différents types de "Kraton", un copolymère tel que "Vector 4113" de Dexco ou "Cariflex S-1707" de Shell.

La résine de forte activité peut être, au lieu de "Dercolyte", la "Piccolyte" S ou HM de Hercules, ou le "Zonatac" de Arizona/Bergvik.

La résine renforçante peut être, au lieu de "Kristalex", une résine "Piccotex" de Hercules ou "LX-685" de Neville.

La résine de faible activité peut être, au lieu de "Regalite", la résine "Arkon P" de Arakawa.

Le plastifiant peut être, au lieu de "Durasyn", une paraffine, une huile de ricin, une cire de paraffine de synthèse de Shell, la résine "Sun 5512" de Sun ou "Primol" de Hercules.

Les hydrocolloïdes peuvent contenir diverses substances telles que les xanthanes, les alginates, la pectine, la gélatine, le psyllium, l'extrait de caroube, la gomme arabique, l'agarose, les carraghénines, des polyacrylamides et autres.

L'agent à action médicinale peut être, au lieu du chitosan, la vitamine E, l'aloe vera, le collagène, l'allantoïne, un extrait d'arnica, la vitamine B5, une échinacée ou autre.

Enfin, il est parfois avantageux d'incorporer un antioxydant, tel que "Irganox" 1010 de Ciba.

On considère maintenant d'autres propriétés des compositions selon l'invention.

### Essai de cytotoxicité

Un essai de cytotoxicité a été réalisé sur deux compositions adhésives selon l'invention et quatre compositions adhésives de poches de recueil de fluides corporels du commerce ("Alterna +", "Moderma Flex", "Nuance light nova" et "Esteem").

L'essai est effectué simultanément pour toutes les compositions, dans les mêmes conditions et sur une même préparation contenant des cellules de peau humaine de même souche. Compte tenu de la dispersion des résultats de cet essai, il convient d'éliminer les valeurs aberrantes et les valeurs extrêmes. Les résultats sont indiqués sous forme d'un taux de destruction des cellules. Plus la valeur (négative) est élevée et plus la composition est agressive vis-à-vis de la peau.

Les deux compositions selon l'invention ont donné respectivement des valeurs de -12 % et -14,5 %. Les quatre compositions de produits connus ont donné des résultats de -17 %, -27 %, -18 % et -34 %.

Etant donné qu'on considère qu'un taux de variabilité de 5 % dans l'essai de cytotoxicité est significatif, il est clair que les compositions adhésives selon l'invention sont moins agressives que les quatre compositions du commerce.

### Essais de tolérance par le patient

Des essais ont été réalisés dans des conditions de milieu hospitalier, c'est-à-dire dans lesquelles la pose et l'enlèvement sont effectués par du personnel hospitalier. Le personnel hospitalier a déterminé la commodité de mise en place et d'enlèvement et la présence éventuelle de fuites. Les patients ont évalué eux-mêmes le confort obtenu, en terme d'oubli ou non de la présence de la poche, et éventuellement des problèmes de d'irritation.

Les résultats indiquent que les éléments formés des compositions selon l'invention simplifient beaucoup la mise en place et l'enlèvement, grâce à la très grande souplesse de la composition. Les patients oublient presque constamment la présence de la poche de recueil de fluides corporels.

On attribue ces résultats non seulement à la qualité de l'adhésivité obtenue, mais aussi à la très grande souplesse et à l'absence d'irritation. En particulier, la soudure réalisée entre un élément adhésif muni d'un film de polyéthylène et la poche autour du trou formé dans l'élément adhésif, ne réduit pas la très grande souplesse et améliore le confort du patient.

Ainsi, l'invention concerne des compositions adhésives et des éléments de fixation qui présentent une excellente adaptabilité grâce à leur très grande souplesse, qui constitue un avantage considérable aussi bien lors de la pose et de l'enlèvement de la composition que par l'oubli de sa présence.

## Revendications

1. Composition adhésive destinée à assurer la fixation sur la peau humaine, **caractérisée en ce qu'**elle contient 55 à 75 % d'une phase continue et 25 à 45 % d'une phase discontinue constituée de polymères hydrophiles sous forme d'hydrocolloïdes, la phase continue contenant 5 à 10 % d'un copolymère de texture constitué d'un copolymère séquencé de type styrène-isoprène-styrène ou styrène-butadiène-styrène ayant une majorité de séquences triples au moins, plus de 22 % d'agent d'adhésivité, et 10 à 30 % d'un plastifiant, ces pourcentages étant indiqués par rapport à l'ensemble de la composition.

2. Composition selon la revendication 1, **caractérisée en ce que** la phase continue contient au maximum 40 % d'agent d'adhésivité, et au maximum 28 % d'un plastifiant, ces pourcentages étant indiqués par rapport à l'ensemble de la composition.

3. Composition selon la revendication 2, **caractérisée en ce que** la phase continue contient 6 à 10 % d'un copolymère de texture, 23 à 38 % d'agent d'adhésivité, et 13 à 25 % d'un plastifiant, ces pourcentages étant indiqués par rapport à l'ensemble de la composition.

4. Composition selon l'une quelconque des revendications 1 à 3, **caractérisée en ce que** l'agent d'adhésivité est un mélange d'agents d'adhésivité qui contient une majorité d'agents d'adhésivité de forte activité, à base de résines terpéniques, et une quantité significative d'un agent d'adhésivité de faible activité d'adhésivité mais présentant en outre une action renforçante.

5. Composition selon la revendication 4, **caractérisée en ce que** la moitié au moins de l'agent d'adhésivité est constituée d'un agent d'adhésivité de forte activité, à base de résine polyterpénique.

6. Composition selon l'une quelconque des revendications 3 et 4, **caractérisée en ce que** le tiers au maximum de l'agent d'adhésivité est un agent d'adhésivité de faible activité constituant une résine renforçante.

7. Composition selon l'une quelconque des revendications précédentes, **caractérisée en ce que** le copolymère de texture de la phase continue est un copolymère séquencé de styrène-isoprène-styrène composé au moins en majorité de séquences au moins triples, les séquences doubles ne constituant au plus qu'une minorité du copolymère.

8. Composition selon l'une quelconque des revendications précédentes, **caractérisée en ce que** la phase discontinue constituée de polymères hydrophiles sous forme d'hydrocolloïdes contient une quantité importante d'au moins un composé choisi parmi les fibres de cellulose, la carboxyméthylcellulose, l'hydroxyéthylcellulose, les composés analogues à la gomme guar, et les alginates.

9. Composition selon l'une quelconque des revendications précédentes, **caractérisée en ce qu'**elle comporte aussi un agent à action médicinale.

10. Elément de fixation de poche de recueil de fluides corporels, **caractérisé en ce qu'**il comprend la composition adhésive selon l'une quelconque des revendications précédentes et, sur la face opposée à celle qui est destinée à être au contact de la peau, un film présentant une propriété de transmission unidirectionnelle d'eau.

11. Elément selon la revendication 10, **caractérisé en ce que** le film est un film de polyéthylène à microperforations conformées facilitant le passage de l'eau dans un sens mais pas dans l'autre.

## Claims

1. Adhesive composition for the fixation on a human skin, **characterized in that** it contains 55-75% of a continuous phase and 25-45% of a discontinuous phase constituted of hydrophilic polymers in the form of hydrocolloids, the continuous phase containing 5-10% of a texturing copolymer constituted of a block copolymer such as styrene-isoprene-styrene or styrenebutadiene-styrene with a majority of triblocks at least, more than 22% of a tackifier, and 10-30% of a plasticizer, these percentages being indicated against the entire composition.

2. Composition according to claim 1, **characterized in that** the continuous phase contains at the most 40% of a tackifier, and at the most 28% of a plasticizer, these percentages being indicated against the entire composition.

3. Composition according to claim 2, **characterized in that** the continuous phase contains 6-10% of a texturing copolymer, 23-38% of a tackifier, and 13-25% of a plasticizer, these percentages being indicated against the entire composition.

4. Composition according to any one of the claims 1 to 3, **characterized in that** the tackifier is a mixture of tackifiers containing a majority of terpene-resin-based tackifiers with a strong activity and a significant amount of a tackifier with a low tackifying activity but having moreover a strengthening effect.

5. Composition according to claim 4, **characterized in that** at least half the tackifier is constituted of a polyterpene-resin-based tackifier with a strong activity.

6. Composition according to any one of the claims 3 and 4, **characterized in that** at the most a third of the tackifier is a tackifier with a low activity forming a strengthening resin.

7. Composition according to any one of the preceding claims, **characterized in that** the texturing copolymer of the continuous phase is a block copolymer of styrene-isoprene-styrene composed of at least a majority of triblocks at least, the diblocks being at the most only a minority of the copolymer.

8. Composition according to any one of the preceding claims, **characterized in that** the discontinuous phase constituted of hydrophilic polymers in the form of hydrocolloids contains an important amount of at least a component selected among cellulose fibers, the carboxymethylcellulose, the hydroxyethylcellulose, the components similar to gum guar, and alginates.

9. Composition according to any one of the preceding claims, **characterized in that** it also comprises an agent with a medicinal effect.

10. Element for the fixation of a pocket receiving body fluids, **characterized in that** it comprises the adhesive composition according to any one of the preceding claims and, on the face opposite the face intended to be in contact with a skin, a film having a property of transmitting water in only one direction.

11. Element according to claim 10, **characterized in that** the film is a film of polyethylene with configured microperforations for facilitating the passage of water in one direction but not in the other.

## Patentansprüche

1. Klebende Zusammensetzung für die Befestigung an eine Menschenhaut, **dadurch gekennzeichnet, dass** sie 55-75% einer kontinuierlichen Phase und 25-45% einer diskontinuierlichen Phase aufweist, die aus hydrophilen Polymeren in der Form von Hydrokolloiden zusammengesetzt ist, wobei die kontinuierliche Phase 5-10% eines texturierenden Copolymers, zusammengesetzt aus einem Blockcopolymer wie Styrol-Isopren-Styrol oder Styrol-Butadien-Styrol mit mindestens einer Mehrzahl von Triblocken, mehr als 22% eines Haftvermögensmittels, und 10-30% eines Plastiziermittels enthält, wobei diese Prozentgehalte auf die gesamte Zusammensetzung bezogen sind.

2. Zusammensetzung nach Anspruch 1, **dadurch gekennzeichnet, dass** die kontinuierliche Phase höchstens 40% eines Haftvermögensmittels und höchstens 28% eines Plastiziermittels enthält, wobei diese Prozentgehalte auf die gesamte Zusammensetzung bezogen sind.

3. Zusammensetzung nach Anspruch 2, **dadurch gekennzeichnet, dass** die kontinuierliche Phase 6-10% eines texturierenden Copolymers, 23-38% eines Haftvermögensmittels und 13-25% eines Plastiziermittels enthält, wobei diese Prozentgehalte auf die gesamte Zusammensetzung bezogen sind.

4. Zusammensetzung nach einem der Ansprüche 1 bis 3, **dadurch gekennzeichnet, dass** das Haftvermögensmittel ein Gemisch von Haftvermögensmitteln ist, das eine Mehrzahl von Haftvermögensmitteln auf Basis von Terpenharzen und mit einer strengen Aktivität und eine bezeichnende Menge eines Haftvermögensmittels mit einer geringen Haftvermögensaktivität aber außerdem mit einer Verstärkungseffekt enthält.

5. Zusammensetzung nach Anspruch 4, **dadurch gekennzeichnet, dass** mindestens die Hälfte des Haftvermögensmittels aus einem Haftvermögensmittel mit einer strengen Aktivität und auf Basis von einem Polyterpenharz zusammengesetzt ist.

6. Zusammensetzung nach einem der Ansprüche 3 und 4, **dadurch gekennzeichnet, dass** höchstens ein Drittel des Haftvermögensmittels in ein ein Verstärkungsharz bildendes Haftvermögensmittel mit einer geringen Aktivität besteht.

7. Zusammensetzung nach einem der vorhergehenden Ansprüche, **dadurch gekennzeichnet, dass** das texturierende Copolymer der kontinuierlichen Phase in ein Blockcopolymer aus Styrol-Isopren-Styrol besteht, das mindestens aus einer Mehrheit von Triblöcken zusammengesetzt ist, wobei die Diblöcke höchstens nur eine Minderzahl des Copolymers bilden.

8. Zusammensetzung nach einem der vorhergehenden Ansprüche, **dadurch gekennzeichnet, dass** die diskontinuierliche Phase, bestehend in hydrophilen Polymeren in der Form von Hydrokolloiden, eine große Menge von mindestens einer Verbindung enthält, die unter Zellfasern, der Carboxymethylzellulose, der Hydroxyethylzellulose, den Verbindungen ähnlich wie Guaran und Alginaten ausgewählt ist.

9. Zusammensetzung nach einem der vorhergehenden Ansprüche, **dadurch gekennzeichnet, dass** sie ebenfalls ein Mittel mit einem Medizinaleffekt aufweist.

10. Element für die Befestigung einer Tasche für die Sammelung von körperlichen Flüssigkeiten, **dadurch gekennzeichnet, dass** es die klebende Zusammensetzung nach einem der vorhergehenden Ansprüche und, auf der der in Berührung mit einer Haut kommenden Seite gegenüberliegenden Seite, eine dünne Folie mit der Eigenschaft des Einrichtungsdurchflusses des Wassers aufweist.

11. Element nach Anspruch 10, **dadurch gekennzeichnet, dass** die dünne Folie in eine Folie von Polyethylen mit aufgeformten Mikroperforationen besteht, die den Durchfluss des Wassers in einer, aber nicht in der anderen Richtung erleichtern.
